# EUROPÄISCHE PATENTSCHRIFT

(11) **EP 0 949 252 B1**
(45) Veröffentlichungstag und Bekanntmachung des Hinweises auf die Patenterteilung: **25.04.2001**
(21) Anmeldenummer: 99106605.1
(22) Anmeldetag: 31.03.1999
(51) Int. Cl.: C07D 251/28, B29C 47/80, B29C 47/86, B30B 11/26

(54) **Verfahren zur Herstellung von Cyanurchlorid-Formlingen**
Process for the production of shaped bodies of cyanuric chloride
Procédé pour la fabrication de pièces moulées de chlorure de cyanuryle

(30) Priorität: 09.04.1998 DE 19816026
(43) Veröffentlichungstag der Anmeldung: 13.10.1999
(73) Patentinhaber: Degussa-Hüls Aktiengesellschaft, 60287 Frankfurt am Main (DE)
(72) Erfinder: Ehrhardt, Knut, 63454 Hanau (DE); Goedecke, Ralf Dr., 63517 Rodenbach (DE); Möller, Rolf Dieter, 64832 Babenhausen (DE); Garcia, Juan, 61137 Schöneck (DE)

(56) Entgegenhaltungen:
- EP-A- 0 036 943
- DE-A- 2 843 379
- DE-A- 19 642 449
- DE-B- 1 266 308
- US-A- 4 591 493

## Beschreibung

Die Erfindung betrifft ein Verfahren zur Herstellung von Cyanurchlorid-Formlingen, wobei die Formlinge stäbchen- oder schuppenförmig sind, durch Abkühlen von Cyanurchloridschmelze in einem formgebenden Werkzeug.

Es ist bekannt, durch Trimerisation von Chlorcyan gewonnenes dampfförmiges Cyanurchlorid direkt oder über flüssiges Cyanurchlorid in festes Cyanurchlorid in feinteiliger Form zu überführen:

Die Abscheidung von pulverförmigem Cyanurchlorid durch Desublimation von dampfförmigem Cyanurchlorid kann in von außen gekühlten Räumen erfolgen oder durch Einleiten des Cyanurchloriddampfes mit einem Inertgas und/oder einer bei dem Abscheidevorgang verdampfenden indifferenten Kühlflüssigkeit in eine Abscheidekammer - siehe beispielsweise DE-PS 12 66 308 und US-PS 4,591,493. Bei der Gewinnung von feinteiligem Cyanurchlorid aus flüssigem Cyanurchlorid wird letzteres in eine Abscheidekammer eingedüst und mit im Kreis geführten inerten Kühlgasen oder durch indirekte Kühlung in der Abscheidekammer abgekühlt, bis sich die Sprühtröpfchen in kristalliner Form abscheiden - siehe beispielsweise DE 28 43 379. Gemeinsam ist den Verfahren ein erheblicher technischer Aufwand für Abscheidekammern und Vorrichtungen zum Rückführen und Reinigen von Prozeß- und Abgasen.

Bei den zuvor gewürdigten sowie auf den gleichen Prinzipien beruhenden Verfahren wird Cyanurchlorid stets in feinteiliger Form, im allgemeinen mit einem maximalen Korndurchmesser von im wesentlichen kleiner 250 µm, erhalten. Derart feinteilige Produkte sind zwar vorteilhaft hinsichtlich ihrer hohen Reaktivität, sie weisen aber eine Reihe von Nachteilen auf, welche für viele Zwecke eine andere Produktform wünschenswert machen.

Die Handhabung, wie Förderung, Lagerung und Dosierung, von feinteiligem Cyanurchlorid bereitet besondere Probleme, weil zur üblichen Staubbildung feinteiliger Stoffe, welche Absaugvorrichtungen erforderlich machen, die korrosiven und reizenden Eigenschaften kommen. Zusätzlich ist Cyanurchlorid hydrolyseempfindlich, wobei dabei gebildete Hydrolyseprodukte nicht nur Cyanurchlorid selbst, sondern auch daraus hergestellte Folgeprodukte verunreinigen können. Feinteiliges Cyanurchlorid ist wegen seiner hohen Oberfläche der Hydrolyse besonders stark zugänglich. Damit kommt es auch leicht zu festen Ablagerungen in den Entstaubungsvorrichtungen und staubführenden Leitungen. Zur Vermeidung und Behebung aufgetretener Störungen sind technisch aufwendige Maßnahmen beziehungsweise Einrichtungen nötig.

Ein weiterer Nachteil feinteiligen Cyanurchlorids ist die unbefriedigende Rieselfähigkeit. Letztere läßt sich zwar durch die Zumischung von Rieselhilfsmitteln, etwa Kieselsäuren, verbessern, jedoch mindert das Rieselhilfsmittel die Produktreinheit des Cyanurchlorids und gegebenenfalls auch der daraus hergestellten Produkte. Gemäß EP-A 0 416 584 läßt sich die Fließfähigkeit von durch Desublimation oder Sprühkristallisation hergestelltem festen Cyanurchlorid auch ohne Zugabe eines Rieselhilfsmittels durch eine Scherbehandlung desselben in einem Kneter oder Mischer, insbesondere bei 60 bis 120 °C, verbessern; die Feinpulvrigkeit des Cyanurchlorids wird aber bei diesem Verfahren nicht beseitigt, denn die mittlere Korngröße beispielhafter Ausführungsformen liegt im Bereich von etwa 10 bis 40 µm.

Die noch nicht veröffentlichte DE-Patentanmeldung 196 42 449.6 lehrt schuppen- und pastillenförmige Cyanurchlorid-Formlinge. Herstellbar sind diese Formlinge durch tropfen- oder streifenförmiges Aufbringen von geschmolzenem Cyanurchlorid auf eine Fläche und Abführen der Schmelzwärme durch Kühlung der Fläche oder Kontaktieren der auf die Fläche aufgebrachten Schmelze mit einem Kühlgas. Ein Nachteil dieses Verfahrens ist, daß die Vorrichtung mit dem Kühlband in gekapselter Form ausgebildet werden muß. Zudem kommt es wegen des hohen Sublimationsdampfdrucks von Cyanurchlorid in der Nähe des Erstarrungspunktes und auch noch oberhalb von 100 °C in erheblichem Umfang zur Sublimation und damit Bildung von feinteiligem Material.

Aufgabe der vorliegenden Erfindung ist demgemäß die Bereitstellung eines weiteren Verfahrens zur Herstellung von Cyanurchlorid-Formlingen. Das Verfahren sollte einfach handhabbar sein; zudem sollte das Verfahren so betrieben werden können, daß es zu keiner nennenswerten Sublimation kommt.

Gefunden wurde ein Verfahren zur Herstellung von Cyanurchlorid-Formlingen durch Abkühlen einer Cyanurchloridschmelze in einem formgebenden Werkzeug, das dadurch gekennzeichnet ist, daß man eine mit einem zur Strangbildung befähigten ein- oder mehrkanaligen Mundstück ausgestattete Druckzelle mit Cyanurchloridschmelze befüllt, die Wandung der Druckzelle und/oder des Mundstücks derart abkühlt, daß am Beginn oder innerhalb der Kanäle des Mundstücks, deren öffnungsquerschnitt über die Kanallänge hinweg im wesentlichen konstant ist, der Schmelzpunkt von Cyanurchlorid unterschritten und am Austritt eine Temperatur von 140 °C oder weniger erreicht wird, durch Krafteinwirkung auf die Druckzelle erstarrtes Cyanurchlorid strangförmig aus dem Mundstück preßt und den Strang anschließend zerkleinert.

Die Unteransprüche richten sich auf bevorzugte Ausführungsformen des Verfahrens.

Die zu verwendende Druckzelle kann in beliebiger Weise ausgebildet sein, beispielsweise als druckbeaufschlagter Vorratsbehälter oder eine mit einem solchen Behälter in Verbindung stehende kühlbare Zelle mit darin integriertem oder angesetzten Mundstück. Die Druckzelle ist mit einem kühlbaren Mundstück mit einem oder mehreren darin befindlichen ungekrümmten Kanälen, deren Öffnungsquerschnitt über die Länge der Kanäle hinweg im wesentlichen gleich ist, ausgestattet. Der Begriff "im wesentlichen" bedeutet, daß der Kanal im Eingangsbereich breiter sein kann, z. B. um Kantenabrieb zu vermeiden oder das Einführen eines Stempels zu erleichtern. Das Mundstück ist über die Außenwand und/oder im Mundstück befindliche Kühlkanäle kühlbar, um die Schmelzwärme und latente Wärme des Cyanurchlorids abzuführen. Es ist ein erfindungswesentliches Merkmal des Verfahrens, daß die Erstarrung des Cyanurchlorids erst in dem oder den Kanälen des Mundstücks, also nach endgültiger Formgebung, erfolgt und das so erstarrte Material unter weiterer Abkühlung aus dem Mundstück gepreßt wird. Die Kanäle müssen vom druckzellenseitigen Eingang bis zum Austritt der Stränge gleichförmig ausgebildet sein, weil sich eine plastische Formgebung nach dem Erstarren des Cyanurchlorids als nicht möglich erwies. Die Verwendung eines Extruders erwies sich wegen der fehlenden plastischen Verformbarkeit des erstarrten Cyanurchlorids einerseits und niedrigen Viskosität der Cyanurchloridschmelze und damit ungenügenden Druckaufbaus andererseits als nicht geeignet.

Die Länge des Mundstücks und/oder die Intensität der Kühlung desselben sind für die Temperatur des/der austretenden Stränge verantwortlich. Durch eine Verlängerung und/oder Intensivierung der Kühlung wird eine niedrigere Austrittstemperatur und damit ein niedrigerer Sublimationsdampfdruck des erstarrten Materials erzielt. Der Sublimationsdampfdruck sinkt von etwa 27 kPa bei 150 °C auf z. B. 6,3 kPa bei 120 °C und 0,27 kPa bei 60 °C.

Vorzugsweise liegt die Austrittstemperatur unter 140 °C, insbesondere unter 100 °C und besonders bevorzugt im Bereich von 40 bis 60 °C. Mit zunehmender Länge nimmt der erforderliche Preßdruck zum Auspressen zu. Mit zunehmender Vorschubgeschwindigkeit nimmt der Preßdruck ab, jedoch steigt die Austrittstemperatur an.

Der Querschnitt des einen oder der mehreren Kanäle im Mundstück kann beliebig sein, bevorzugt wird aber ein runder, ovaler oder rechteckiger Querschnitt. Bei rundem Querschnitt liegt der Durchmesser im allgemeinen im Bereich von 1 bis 5 mm; Kanäle mit rechteckigem Querschnitt, also insbesondere spaltförmige Kanäle, sind bevorzugt 5 bis 30 mm breit und 1 bis 5 mm hoch.

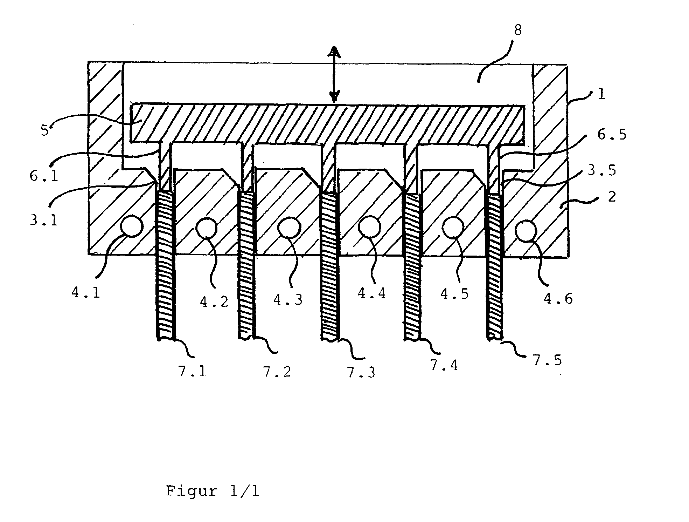

Die Figur zeigt in schematischer Form einen Querschnitt einer besonders zweckmäßigen Ausführungsform des Unterteils einer Druckzelle mit einem 5-kanaligen Mundstück: Am Boden der Druckzelle 1 ist das Mundstück 2 mit den Kanälen 3.1 bis 3.5 angeordnet. Das Mundstück umfaßt eine Kühlzone mit Kühlkanälen 4.1 bis 4.6. In der Druckzelle ist eine Stempelplatte 5 mit daran fixierten, in die Kanäle passenden Nadeln 6.1 bis 6.5 vertikal beweglich angeordnet (Hubgestänge nicht dargestellt). Die Nadeln 6.1 bis 6.5 reichen in der tiefsten Stellung des Stempels 5 nur teilweise in die Kanäle 3.1 bis 3.5 - der untere Teil der Kanäle bleibt mit erstarrten Cyanurchloridsträngen 7.1 bis 7.5 verschlossen, damit die Cyanurchloridschmelze 8 nicht aus der Druckzelle auslaufen kann.

Den zum Auspressen erforderlichen Druck kann der Fachmann in beliebiger Weise aufbauen. Beispielsweise kommen hierfür Hochdruckkolbenpumpen und Membranpumpen in Betracht. Es ist auch möglich, den Druckaufbau und damit das Auspressen des/der Stränge durch Einführung eines Druckstempels mittels einer Spindelpresse in einen entsprechenden Zylinder oder unmittelbar in den oder die Kanäle des Mundstücks zu bewirken. Die Verwendung eines druckbeaufschlagten Vorratsbehälters, mit welchem die eigentliche Zelle mit dem Mundstück in Verbindung steht, wird bevorzugt. Das Preßverfahren kann taktweise oder kontinuierlich durchgeführt werden. Der Auspreßdruck liegt je nach der Länge des Mundstücks und der gewünschten Vorschubgeschwindigkeit im allgemeinen im Bereich von 1 MPa bis 100 MPa, insbesondere 1 MPa bis 20 MPa und besonders bevorzugt 1 bis 5 MPa.

Die Zerkleinerung des oder der aus dem Mundstück austretenden Stränge oder Bänder in Stücke, insbesondere solche mit einer Länge im Bereich von 5 bis 30 mm kann unter Verwendung üblicher Brech- oder Schneidewerkzeuge erfolgen.

Erfindungsgemäß hergestellte Cyanurchlorid-Formlinge sind durchkristallisiert, lager- und transportfähig sowie weitgehend staubfrei. Das erfindungsgemäße Verfahren zeichnet sich dadurch aus, daß die Erstarrung der Cyanurchloridschmelze und weitere Abkühlung des Feststoffs in einem geschlossenen Kanal erfolgen, wodurch Probleme, wie Bildung von Desublimat während des Erstarrens und Abkühlens, weitgehend vermieden werden.

### Beispiele

In einer Laboranlage wurden Cyanurchlorid-Strangpreßlinge hergestellt. Die hierzu verwendete Vorrichtung umfaßte eine kühlbare zylinderförmige Druckzelle aus abrasionsbeständigem Werkstoff mit im Unterteil integriertem kapillarförmigem Mundstück. Der Kapillarendurchmesser betrug 5 mm. Die Zelle befand sich in einem Druckzellenfuß mit einer Kammer zur Aufnahme des ausgetragenen Strangs. Zum Auspressen wurde eine Drucknadel mittels einer Spindelpresse in die Zelle eingeführt. Variiert wurden die Vorschubgeschwindigkeit und Länge der Kapillare. Der Austragsdruck wurde registriert.

| Länge der Kapillare (mm) | Vorschub (mm/min) | Maximaldruck beim Austrag (MPa) |
|---|---|---|
| 20 | 5 | 3 |
| 30 | 5 | 14 |
| 40 | 5 | 62 |
| 50 | 5 | 170 |
| 20 | 60 | 11 |
| 20 | 1000 | 2,5 |
| 20 | 1600 | 2,5 |

Die Temperatur des ausgetragenen Strangs lag in allen Fällen um/unter 60 °C.

## Patentansprüche

1. Verfahren zur Herstellung von Cyanurchlorid-Formlingen durch Abkühlen einer Cyanurchloridschmelze in einem formgebenden Werkzeug,
dadurch gekennzeichnet,
daß man eine mit einem zur Strangbildung befähigten ein- oder mehrkanaligen Mundstück ausgestattete Druckzelle mit Cyanurchloridschmelze befüllt, die Wandung der Druckzelle und/oder des Mundstücks derart abkühlt, daß am Beginn oder innerhalb der Kanäle des Mundstücks, deren Öffnungsquerschnitt über die Kanallänge hinweg im wesentlichen konstant ist, der Schmelzpunkt von Cyanurchlorid unterschritten und am Austritt eine Temperatur von 140 °C oder weniger erreicht wird, durch Krafteinwirkung auf die Druckzelle erstarrtes Cyanurchlorid strangförmig aus dem Mundstück preßt und den Strang anschließend zerkleinert.

2. Verfahren nach Anspruch 1,
dadurch gekennzeichnet,
daß die Kanäle des Mundstücks einen runden, ovalen oder rechteckigen Öffnungsquerschnitt aufweisen,
insbesondere einen runden Öffnungsquerschnitt mit einem Durchmesser im Bereich von 1 bis 5 mm oder einen rechteckigen Öffnungsquerschnitt mit einer Breite im Bereich von 5 bis 30 mm und einer Höhe im Bereich von 1 bis 5 mm.

3. Verfahren nach Anspruch 1 oder 2,
dadurch gekennzeichnet,
daß die Krafteinwirkung auf die Druckzelle durch Durchbeaufschlagung eines mit der Druckzelle in Verbindung stehenden Vorratsbehälters oder mittels einer Hochdruckkolbenpumpe oder durch periodisches Einbringen eines Stempels in die Kanäle erfolgt.

4. Verfahren nach einem der Ansprüche 1 bis 3,
dadurch gekennzeichnet,
daß man das Mundstück derart abkühlt, daß erstarrtes Cyanurchlorid mit einer Temperatur im Bereich von 40 bis 60 °C austritt.

5. Verfahren nach einem der Ansprüche 1 bis 4,
dadurch gekennzeichnet,
daß man erstarrtes Cyanurchlorid bei einem Druck von 1 bis 20 Mpa, insbesondere 1 bis 5 MPa, aus der Druckzelle preßt.

## Claims

1. Process for the production of cyanuric chloride mouldings by cooling a cyanuric chloride melt in a moulding tool,
characterized in that
a pressure cell equipped with a single or multi-channel die which is capable of strand formation is filled with cyanuric chloride melt, the wall of the pressure cell and/or the die is cooled in such a way that at the start of or within the channels of the die, the orifice cross-section of which is substantially constant over the channel length, the temperature is below the melting point of cyanuric chloride and a temperature of 140 °C or less is reached at the outlet, solidified cyanuric chloride is pressed out of the die in strand form by the effect of force on the pressure cell and the strand is then reduced in size.

2. Process according to claim 1,
characterized in that
the channels of the die have a round, oval or rectangular orifice cross section,
particularly a round orifice cross section with a diameter in the range from 1 to 5 mm or a rectangular orifice cross section with a width in the range from 5 to 30 mm and a height in the range from 1 to 5 mm.

3. Process according to claim 1 or 2,
characterized in that
the effect of force on the pressure cell takes place by applying pressure to a feed container connected to the pressure cell or by means of a high pressure piston pump or by periodic introduction of a ram into the channels.

4. Process according to one of claims 1 to 3,
characterized in that
the die is cooled in such a way that solidified cyanuric chloride with a temperature in the range from 40 to 60 °C emerges.

5. Process according to one of claims 1 to 4,
characterized in that
solidified cyanuric chloride is pressed out of the pressure cell at a pressure of 1 to 20 MPa, particularly 1 to 5 MPa.

## Revendications

1. Procédé de production de pièces moulées de chlorure de cyanure par refroidissement d'un produit de fusion de chlorure de cyanure dans un outillage qui donne une forme,
caractérisé en ce qu'
on remplit une cellule de pression équipée d'une embouchure à un ou plusieurs canaux capables de former des filaments, d'un produit de fusion du chlorure de cyanure, on refroidit la paroi de la cellule de pression et/ou de l'embouchure de telle sorte qu'au début ou à l'intérieur des canaux de l'embouchure dont la section d'ouverture est constante tout au long de la longueur du canal, on passe en-dessous du point de fusion du chlorure de cyanure et qu'à la sortie on atteint une température de 140°C ou moins, on presse par action d'une force sur la cellule de pression le chlorure de cyanure solidifié sous forme de filaments et on fragmente ensuite le filament.

2. Procédé selon la revendication 1,
caractérisé en ce que
les canaux de l'embouchure possèdent une section d'ouverture ronde, ovale, rectangulaire, en particulier une section transversale d'ouverture ronde, ayant un diamètre dans la zone de 1 à 5 mm ou une section transversale d'ouverture rectangulaire ayant une largeur dans la zone de 5 à 30 mm et une hauteur dans la zone de 1 à 5 mm.

3. Procédé selon la revendication 1 ou 2,
caractérisé en ce que
l'action de la force sur la cellule de pression s'effectue par alimentation d'un récipient d'alimentation qui se tient en liaison avec la cellule de pression, ou à l'aide d'une pompe à piston à haute pression ou par introduction périodique d'un poinçon dans les canaux.

4. Procédé selon l'une quelconque des revendications 1 à 3,
caractérisé en ce qu'
on refroidit l'embouchure de telle sorte que le chlorure de cyanure solidifié sorte à une température dans la zone de 40 à 60°C.

5. Procédé selon l'une quelconque des revendications 1 à 4,
caractérisé en ce qu'
on presse le chlorure de cyanure solidifié, à une pression de 1 à 20 Mpa, en particulier 1 à 5 Mpa, vers l'extérieur de la cellule de pression.
